Europäisches Patentamt

European Patent Office

Office européen des brevets

(11) Publication number: **0 379 347 A2**

# EUROPEAN PATENT APPLICATION

(21) Application number: **90300467.9**

(22) Date of filing: **17.01.90**

(51) Int. Cl.5: **C12Q 1/28, G01N 33/535, C12Q 1/00, G01N 33/58, G01N 33/76**

(30) Priority: **17.01.89 US 298099**

(43) Date of publication of application:
**25.07.90 Bulletin 90/30**

(84) Designated Contracting States:
**AT BE CH DE DK FR GB IT LI LU NL SE**

(71) Applicant: **EASTMAN KODAK COMPANY**
**343 State Street**
**Rochester, New York 14650(US)**

(72) Inventor: **Warren, Harold Chester III, C/o**
**Eastman Kodak Comp**
**Patent Department, 343 State Street**
**Rochester, New York 14650(US)**
Inventor: **Norkus, Norbert Sarunas, C/o.**
**Eastman Kodak Comp.**
**Patent Department, 343 State Street**
**Rochestester, New York 14650(US)**
Inventor: **Smith-Lewis, Margaret J. C/o.**
**Eastman Kodak Comp.**
**Patent Department, 343 State Street**
**Rochester, New York 14650(US)**

(74) Representative: **Nunney, Ronald Frederick**
**Adolphe et al**
**Kodak Limited Patent Department Headstone**
**Drive**
**Harrow Middlesex HA1 4TY(GB)**

(54) **Enzyme-labeled receptor composition, diagnostic kit and use in method to determine a ligand.**

(57) An aqueous reagent composition has a pH in the range of from 7 to 9 and comprises: an enzyme-labeled receptor to a ligand of interest, and an organic buffer having a pKa of from 5 to 7 at 25°C which is present in an amount of from 0.1 to 1 molar. This composition can be combined with a composition which provides a dye in the presence of the enzyme and an appropriate substrate in a diagnostic test kit useful for ligand determination. Such determination includes contacting a specimen containing the ligand with the reagent composition to form an immunological complex, separating the complex from other materials, and detecting either the complex or the uncomplexed enzyme-labeled receptor.

## ENZYME-LABELED RECEPTOR COMPOSITION, DIAGNOSTIC KIT AND USE IN METHOD TO DETERMINE A LIGAND

The present invention relates to a reagent composition, diagnostic test kit and a method for determination of a ligand in a test specimen, such as biological fluids. It is particularly useful for the determination of hCG as an early indicator of pregnancy.

There is a continuous need in medical practice, research and diagnostic procedures for rapid, accurate, qualitative or quantitative determinations of biological substances which are present in biological fluids at low concentrations. For example, the presence of drugs, narcotics, hormones, steroids, polypeptides, prostaglandins or infectious organisms in blood, urine, saliva, vaginal secretions, seminal fluids and other biological fluids has to be determined in an accurate and rapid fashion for suitable diagnosis or treatment.

To provide such determinations, various methods have been devised for isolating and identifying biological substances employing specific binding reactions between the substance to be detected (identified as a "ligand" herein) and receptors specifically reactive with that substance. Radioactive or enzyme labels have been used to detect the resulting reactive complex.

Analytical methods developed for determination of ligands include competitive immunoassays and enzyme linked immunosorbent assays (ELISA) which use an enzyme label on appropriate receptor molecules for detecting the presence or absence of an immunological complex. Another type of test which has been developed is what is known in the art as an immunometric or a "sandwich" assay. Such an assay involves "sandwiching" the ligand (such as an antigen) with two or more receptor molecules (such as antibodies) which complex with the compound in a noninterfering manner and at different epitopic sites. Examples of such essays are described in U.S. Patent 4,486,530 where monoclonal antibodies having high affinity are used.

A kit useful for the determination of hCG has been marketed since early 1988 by Eastman Kodak as the Kodak SurecellTM hCG-Urine Test Kit. This kit contains various reagents and equipment including a disposable test device for performing the assay rapidly and sensitively. Features of the test kit and performance of the assay are described and claimed in EP-A-0 321 260 and EP-A-0 321 261. The assays described therein are generally carried out in a disposable test device in which some of the reagents (including a biotinylated antibody) are immobilized as small drops on side walls of test wells in the device during the manufacturing process. It is also taught therein that the buffer used to maintain pH 7 to 9 in the assay medium is likewise immobilized on the side walls. The buffer used in such assays is 3-(N-morpholino)propanesulfonic acid (commonly known as MOPS) which has a pKa of 7.2 at 25° C. However, in some cases during the manufacturing process, some of the buffer flows down onto the microporous membranes at the bottom of the test wells because of the large size of the drops and unavoidable vibration by the manufacturing equipment.

If too much buffer collects on the microporous membrane, drainage and washing are impeded and false coloration on the membrane is observed. Moreover, when the buffer flows through the membrane, buffering capacity is lost. It would be an advantage to eliminate this manufacturing step altogether.

It was thus considered to put the MOPS buffer in a reagent solution which would be used early in the assay. Such a reagent solution used in the assays comprises a peroxidase-labeled antibody conjugate. However, the presence of MOPS in this enzyme conjugate solution resulted in a loss of enzyme activity. Since the SurecellTM kit is designed for doctors' offices and therefore requires considerable shelf life, a loss in conjugate stability is highly unacceptable. In addition, higher background signals were observed.

Thus, there was a need to find a way to suitably buffer the assay while avoiding the problems in manufacturing and enzyme activity loss noted above.

The problems noted above are overcome with an aqueous reagent composition having a pH of from 7 to 9 and comprising an enzyme-labeled receptor which is specifically reactive with a ligand of interest, the composition characterized as further comprising an organic buffer having a pKa of from 5 to 7 at 25° C and present in an amount of from 0.1 to 1 molar.

This invention also provides a diagnostic test kit useful for the determination of a ligand comprising:

a. the aqueous reagent composition described above, and

b. a dye-providing composition which provides a dye in the presence of the enzyme and a substrate therefor.

Further, a method for the determination of a ligand comprises:

A. contacting a specimen suspected of containing a ligand of interest with an enzyme-labeled receptor therefor to form an immunological complex, the receptor provided in the aqueous reagent composition described above,

B. separating the complex from uncomplexed enzyme-labeled receptor, and

C. detecting either the complex or uncomplexed enzyme-labeled receptor as an indication of the presence of the ligand in the specimen.

The reagent composition, diagnostic test kit and method of the present invention provide a rapid and accurate means for detecting the presence or absence of a ligand of interest in a fluid sample. Moreover, the present invention advantageously builds upon the advances in the art provided by the inventions described and claimed in EP-A-0 321 260 and EP-A-0 321 261.

More particularly, this invention provides a means for buffering an assay without the need to physically immobilize the buffer on a solid surface which is contacted by the test specimen. For instance, in the noted references, the buffer is applied and dried on the sloping walls of test wells in test devices. This innovation has its advantages, but suffers from certain problems noted above. The present invention avoids them.

These advantages are provided by putting a certain buffer in a reagent composition which also comprises an enzyme-labeled receptor for the ligand of interest. While EP-A-0 321 260 and EP-A-0 321 261 teach the use of MOPS as the assay buffer, it is not used in direct contact with the receptor prior to the assay. When we attempted to put MOPS in the receptor composition, we found that it undesirably destabilizes the enzyme in the enzyme-labeled receptor at the concentrations needed so that the receptor has poor shelf life in solution. Thus, after considerable development effort, we found that certain buffers can be used at concentrations which will not destabilize the enzyme, and will adequately buffer low pH biological samples. Such buffers are those having a pKa of from 5 to 7 at 25°C. Moreover, the buffer is present in an amount of from 0.1 to 1 molar. Further details of such buffers are provided below.

The present invention provides a means for determining a chemical or biological compound of interest (identified as a ligand herein) using specific binding reactions between that ligand and a naturally occurring or synthetic receptor therefor. A receptor will bind specifically to that ligand. The determination can be made by merely detecting the presence or absence of the ligand, or by quantitatively determining the amount of ligand.

In particular, the invention can be used to assay biological fluids of animals, humans or plants, but preferably of humans. Such fluids include, but are not limited to, whole blood, plasma, sera, lymph, bile, urine, spinal fluid, synovial fluid, seminal fluid, lacrimal fluid, vaginal secretions, sputum, perspiration and the like as well as stool specimens. It is also possible to assay fluid preparations of human or animal tissue such as skeletal muscle, heart, kidney, lungs, brains, bone marrow, skin and the like.

The present invention can be used to determine any monovalent, multivalent or multideterminant ligand. A monovalent ligand has a single epitopic site for complexation. A multivalent ligand has two or more epitopic sites for complexing with a multiplicity of the same specific binding receptor. A multideterminant ligand has two or more epitopic sites for complexing with a multiplicity of different receptors.

The ligand of interest can be an immunological species which is (1) any substance which, when presented to an immunocompetent host, will result in the production of a specific antibody capable of binding with that substance, or (2) the antibody so produced, which ligand participates in an antigen-antibody reaction.

Representative ligands detectable with the present invention include primary amines, amino acids, peptides, polypeptides, proteins, lipoproteins, glycoproteins, drugs, haptens, enzymes, steroids, lipids, nucleic acids, oligonucleotides, hormones, vitamins, polysaccharides, glycolipids, alkaloids, organisms (bacteria, protozoa, fungi, viruses including retroviruses, rickettsia and the like) and components thereof, blood components, tissue and organ antigens and other materials known to one skilled in the art. In some instances, the ligand is an antibody which is directed against a drug, hormone, antibiotic or other compound having antigenic properties. Where the ligand is an antigen or antibody and the receptor is an antibody to either of these, either monoclonal or polyclonal antibodies can be used, and they can be whole molecules or various fragments thereof. Preferably, monoclonal antibodies are used in the present invention.

In a preferred embodiment, the invention is useful for the detection of hCG as an early indicator of pregnancy. In this embodiment, one or more different antibodies to hCG are reacted with the antigenic hormone to form an immunological complex which can be detected in a suitable manner. Preferably, at least one of the antibodies is enzyme labeled, and the other is either attached to a solid support material or capable of becoming so attached, such as through an avidin-biotin complex.

The aqueous reagent composition of this invention includes an enzyme-labeled receptor for the ligand of interest. The receptor is readily chosen depending upon what the ligand is, whether it be an antibody, antigenic material or other specific binding material, such as a complementary oligonucleotide (for example, a primer or nucleic acid sequence). Useful enzyme labels are well known in the art and include, but are not limited to, peroxidase, alkaline phosphatase, malate dehydrogenase, glucose oxidase, urease, catalase or glucose-6-phosphate dehydrogenase. Peroxidase is preferred. Enzyme-labeled receptors for many ligands

3

are available commercially. Alternatively, attachment of enzyme to receptor can be accomplished using any of a number of procedures known in the art, for example, that taught by Yoshitake et al, Eur. J. Biochem., 101, 395 (1979) for attaching peroxidase to receptor molecules. The enzyme-labeled receptor can be present in the composition in a wide range of concentration, depending upon the particular receptor, ligand of interest, type of assay, sensitivity needed and other factors that one skilled in the art would be able to evaluate. In most instances, it is present in an amount of from 0.2 to 20 $\mu$g/ml, and preferably from 0.4 to 10 $\mu$g/ml.

In a preferred embodiment, this composition comprises enzyme-labeled antibodies, such as monoclonal antibodies to hCG. The enzyme is preferably peroxidase.

In order to obtain the advantages noted above, the reagent composition comprises one or more buffers which buffer the composition at a pH of from 7 to 9. Each buffer has a pKa of from 5 to 7 at 25° C. Preferably, the buffer pKa is from 5.5 to 6.5 at 25° C.

Useful buffers include, but are not limited to, 2-(N-morpholino)ethanesulfonic acid, tris(hydroxymethyl)-aminomethane, and N-(2-acetamido)-2-aminoethanesulfonic acid, with 2-(N-morpholino)ethanesulfonic acid (MOPS) being preferred.

The amount of buffer present can vary depending upon the type of buffer used and the amount of buffering capacity desired. For example, if the test specimen to be assayed normally has a low pH, more buffering capacity may be needed. Generally, the amount of buffer is from 0.1 to 1, and preferably from 0.1 to 0.5, molar. It is an advantage of this invention that the amount of buffer used in the composition is generally less than that required when MOPS or other higher pKa buffers are used to buffer the test specimen at a pH of 8.

Other optional addenda, such as preservatives, surfactants, nonimmunological proteins (such as casein, bovine serum albumin and others known in the art) can be included in the reagent composition of this invention as long as they do not deleteriously affect the stability of the enzyme-labeled receptor and assay performance.

The reagent composition can be included as part of a diagnostic kit for ligand determination in combination with a dye-providing composition which provides a dye in the presence of the enzyme label and appropriate enzyme substrate. The dye-providing composition then includes one or more reagents which result in a dye upon reaction with the enzyme. Each enzyme has substrate it will catalytically act upon. In some cases, one of the dye-providing reagents is a substrate. In other cases, a separate substrate must be provided.

For example, when peroxidase is used as a label, the kit could also include hydrogen peroxide and appropriate dye-forming reagents, such as a tetramethylbenzidine or a leuco dye which provides a dye in the presence of hydrogen peroxide and peroxidase (for example, a triarylimidazole leuco dye as described in U.S. Patent 4,089,747 or a triarylmethane leuco dye as described in U.S. Patent 4,670,385). A preferred dye-providing composition is described and claimed in EP-A-0 308 236. Useful substrates and dye-forming reagents for other useful enzymes are well within the skill of an ordinary worker in the art.

The kit could also include other reagents, test equipment and instructions for the assay if desired, or each of the materials can be supplied separately. For example, the kit can include a second receptor (such as a second antibody) which is directed to a different epitopic site of the ligand than the labeled receptor, insolubilizing reagents (such as polymeric particles), test devices (described below), pipettes, wash solutions, diluents, extraction compositions, filter devices and other components readily apparent to one skilled in the art.

The device useful in the practice of this invention comprises a water-insoluble substrate which is chemically and immunologically inert (that is, nonreactive) with the ligand, receptor or other reagents used in the assay. The substrate can be a flat test slide, paper, cup, petri dish, test tube, plate, membrane or other suitable configured material which will accommodate one or more reagents and which can be contacted in some manner with a test specimen. Either the specimen can be added to the device, such as a test tube or microtest plate, or the device can be added to it or otherwise contacted for a period of time sufficient for the assay to occur. The device can be disposable or reuseable.

In one embodiment, the device can be used to react ligand and receptor, but the resulting complex is then put into a second device in which the amount of ligand is determined in a suitable fashion. Alternatively, the device can be a device where the assay is carried out, such as a microtest plate having a multiplicity of preformed test wells. Various test devices are known in the art including U.S. Patents 3,825,410, 3,888,629, 3,970,429 and 4,446,232. One useful device is illustrated in EPA-0 308 231.

The device of this invention can be a device whereby the reagents are mixed for later use in an assay in a different device or container. Preferably, the device is designed as a test device for both reagent mixing and testing either in the same or a different location of the device.

More specifically, the test device of this invention comprises a water-insoluble substrate having one or more test zones therein each of which can accommodate a sample of a specimen and appropriate reagents.

The substrate can be prepared from any useful water-insoluble material such as glass, polymeric materials, fibrous materials, cellulosic materials and other materials known in the art.

In a preferred embodiment, the test device has three test zones or wells designed for providing a test result and positive, and negative control results. Such a device would be particularly useful in a doctor's office or in a consumer's home as part of a diagnostic kit, such as a pregnancy test kit. For example, the device described in EP-A-0 308 231 comprises at least one liquid-collecting well (or, as described herein, a test well), means in that compartment defining a vent aperture fluidly connecting the compartment to the atmosphere, and a closure means for shutting off the vent aperture. Preferably, the test device has three separate test wells, one for a test sample, a second for a negative control and a third for a positive control.

The method of this invention is practiced by contacting a specimen suspected of containing a ligand of interest (such as urine containing hCG) with the reagent composition of this invention so as to form an immunological complex between ligand and enzyme-labeled receptor. Preferably, this contact occurs in a test device as described herein.

The complex thus formed is separated from uncomplexed materials in a suitable manner (for example, centrifugation, filtration or by washing uncomplexed materials from immobilized complex). The presence or absence of ligand in the specimen can then be determined by detecting either the complex formed, or the amount of uncomplexed enzyme-labeled receptor. This is usually done by adding suitable substrates and dye-providing reagents as noted above and observing any resulting dye signal using standard procedures and equipment.

Thus, the method can be an ELISA assay in which the ligand is an antigenic material (including haptens) to which receptor antibodies can be generated. Alternatively, the ligand can be an antibody and the receptor can be an antigenic material or an anti-antibody.

Preferably, the assay is an immunometric (or "sandwich") assay in which the ligand is complexed at two or more different epitopic sites with two or more different receptors. One of these receptors is the enzyme-labeled receptor in the reagent composition of this invention. The second receptor is generally either insolubilized by being attached to a solid support material, or is capable of such attachment through a complexing reaction (for example, avidin to biotin or a lectin to a sugar). Attachment of receptor molecules to solid support materials (such as microtiter plates, test tube walls, polymeric particles, and others known in the art) can be done using any of many known procedures and reagents.

Preferably, the second receptor is an antibody which is attached to polymeric particles directly (that is, through adsorption or covalent attachment with or without linking groups), or is biotinylated for complexing with particles having avidin on the surface thereof.

In a preferred embodiment, a method for the determination of hCG comprises:

A. contacting a biological specimen with an unlabeled monoclonal antibody to hCG to form an immunological complex with any hCG present in the specimen,

B. prior to, simultaneously with or subsequent to the contacting in step A, contacting the specimen with an enzyme-labeled monoclonal antibody to hCG to form an immunological complex, the labeled antibody provided in an aqueous reagent composition having a pH of from 7 to 9 and comprising an organic buffer having a pKa of from 5 to 7 at 25°C and present in an amount of from 0.1 to 0.5 molar, the labeled antibody being directed to a different epitopic site than the unlabeled antibody,

C. separating the resulting complex of hCG and the antibodies from uncomplexed materials, and

D. adding a dye-providing composition which will provide a dye in the presence of the enzyme label as an indication of the presence of hCG in the specimen.

In this embodiment, it is particularly desirable that the unlabeled monoclonal antibody be biotinylated for eventual complexation with avidin which is attached to a solid support material (such as polymeric particles). Thus, the resulting complex of hCG and the two antibodies can be readily separated from uncomplexed materials using a microporous filtration membrane or other suitable means.

The following examples are representative of the practice of this invention but are not intended to limit the scope of this invention.

Materials:

Antibody-biotin conjugates were prepared using anti-hCG monoclonal antibodies obtained from Immuno-Search, Inc. and biotin-ω-aminocaproic acid-N-hydroxysuccinimide obtained from Calbiochem-Behring Corp. using the method described by Hofmann et al, J.A.C.S. 100, 3585 (1978).

Human chorionic gonadotropin (hCG) was obtained from Calbiochem.

Antibody-peroxidase conjugates were prepared using anti-hCG monoclonal antibodies obtained from Cambridge Medical Diagnostics and horseradish peroxidase by the method described by Yoshitake et al, Eur. J. Biochem. 101, 395 (1979).

A leuco dye solution was prepared with 2-(4-hydroxy-3,5-dimethoxyphenyl)-4,5-bis(4-methoxyphenyl)-imidazole as follows:

Solid leuco dye (to make a 0.1% solution) was dissolved in a solution of 20% poly(vinyl pyrrolidone) in sodium phosphate buffer (5 mmolar). This solution was then added to a solution containing hydrogen peroxide (10 mmolar), 4'-hydroxyacetanilide electron transfer agent (5 mmolar) and diethylenetriaminepentaacetic acid chelating agent (10 μmolar) in sodium phosphate buffer to produce a final concentration of 1% poly(vinyl pyrrolidone) and 0.005% leuco dye.

Succinylated casein was prepared by reacting case in with an equal weight of succinic anhydride for four hours at 25° C, then purifying the product by dialysis.

MOPS buffer is 3-(N-morpholino)propanesulfonic acid (pKa of 7.2), TRIS is tris(hydroxymethyl)-aminomethane, ACES buffer is N-(2-acetamido)-2-aminoethanesulfonic acid (pKa of 6.8) and MES buffer is 2-(N-morpholino)ethanesulfonic acid (pKa of 6.15).

Examples 1-3: Reagent Composition

Aqueous reagent compositions of the present invention were prepared by mixing: a solution of buffer (50 ml, 0.25 molar, pH 8.2), 4'-hydroxyacetanilide (0.75 g), bovine serum albumin (0.5 g) and thimerosal preservative (0.5 ml of 1 weight % solution). This solution was filtered through a 0.22 μm filter, followed by addition of the peroxidase-labeled monoclonal anti-hCG conjugate described above (0.23 ml, 3.68 mg/ml in phosphate buffered saline solution). The buffer for the compositions were as follows: Example 1, 2-(N-morpholino)ethanesulfonic acid, Example 2, tris(hydroxymethyl)aminomethane, and Example 3, N-(2-acetamido)-2-aminoethanesulfonic acid.

Example 4: Assay of Urine for hCG

Preparation of Insolubilizing Reagent:

The three solutions outlined below were continuously added to a 1365 ml vessel containing deoxygenated water at 80° C at the indicated rates:

Solution 1: Styrene (739 g), m & p-(2-chloroethylsulfonylmethyl)styrene (82 g) and 1-dodecanethiol (8.2 g) at 2.5 g/min. for 380 minutes.

Solution 2: Ammonium persulfate (19.7 g) and distilled, deoxygenated water (1152 g) at 2.14 g/min. for 380 minutes.

Solution 3: Sodium pyrosulfite (9.9 g) and distilled water (1152 g) at 2.27 g/min. for 380 minutes.

After 380 minutes, the reaction was stopped, yielding about 1218 g of latex at 33.4% solids. The latex was dialyzed for 3 days to yield a latex having 27.3% solids and a pH of 5. This latex was diluted to 13.5% solids. NMR analysis confirmed a 96:4 molar ratio of styrene to the second monomer. The resulting latex particles had an average diameter of about 0.67 μm as measured by transmission electron microscopy.

A sample (0.75 ml) of the latex described above was diluted to 20 ml with borate buffer (50 mmolar, pH 8.5) and avidin (5 mg) was subsequently added. The resulting suspension was agitated in an end-over-end fashion at 25° C for 18 hours, followed by centrifugation. The supernatant was discarded and the particles washed once with buffer by centrifugation and resuspended in 10 ml glycine buffer. Biotin binding analysis (that is, titration with tritium labeled biotin) indicated that avidin had been covalently attached to the particles ($7 \times 10^{-6}$ molar binding sites per 0.3% bead suspension) to form a reagent of the present invention.

Assay:

Test devices, similar to those described in EP-A-0 321 261, were used to determine hCG in a urine specimen in the following manner. Each test device comprised: a negative control well to show background and to act as a reference test, a positive control well to indicate that the reagents and procedures were used properly, and a test well for the assay. Each test well contained a filter membrane consisting of a microporous nylon filter membrane.

Control device A was prepared having MOPS buffer immobilized within the test device as described in EP-A-0 321 261. The negative control test well in the device contained MOPS buffer (2 mg) and poly-(acrylamide) binder (60 $\mu$g). The specimen test well contained a dried coating of biotinylated anti-hCG antibodies (3 $\mu$g) immobilized in poly(acrylamide) binder (60 $\mu$g) in one location, and dried MOPS buffer (2 mg) in a different location in the test well. The positive control well contained biotinylated anti-hCG antibodies (3 $\mu$g) immobilized in poly(acrylamide) binder (60 $\mu$g), and dried hCG (400 ml.U.) in one location, and MOPS buffer (2 mg) immobilized in a different location. This test device was used with a reagent composition of Example 2 containing TRIS as buffer.

A similar Control device B was prepared, but no buffer was immobilized in the test device. The device was used with a reagent composition like the Example 1 composition with MOPS used in place of MES as buffer.

A third device was prepared like Control device A but having no buffer immobilized therein. It was used with the reagent composition of Example 1. This device and composition were used in the present Example 4.

All test devices and reagent compositions were kept at 37°C and less than 45% relative humidity for two weeks.

Following this keeping test, the test devices were used to assay urine samples for hCG. These samples had been prefiltered to remove impurities and were known to contain 50 ml.U./ml of hCG. The samples were added to all wells of each test device. The respective reagent compositions were then added to the test devices. After a one minute incubation period, a suspension of the insoluble immunoreactive reagent described above (40 $\mu$l of a 0.45% dispersion) was added to each device and fluid was allowed to drain through the membrane in each well.

A wash solution (200 $\mu$l) comprising sodium phosphate (0.1 molar) and sodium decylsulfate (24 mmolar) was added to each well, followed by the addition of the leuco dye solution described above (40 $\mu$l). After two minutes, the color formed on each membrane was evaluated by reflectance measurements using standard equipment and the results were converted to transmittance density ($D_T$) using the Williams-Clapper transform (J.Opt.Soc.Am., 43, 595, 1953).

The results of the assays are shown in Table I below as the average of three replicates for the determination of 50 ml.U./ml of hCG. The results indicate that only the composition and assay of the present invention (Example 4) exhibited good sensitivity and low background after the keeping test. Neither Control assay using the Control devices and reagent compositions gave adequate sensitivity and low background after the keeping test.

TABLE I

| Assay | $D_T$ | |
| --- | --- | --- |
| | Background | Test Well |
| Control A | 0.012 | 0.031** |
| Control B | 0.028* | 0.041** |
| Example 4 | 0.019 | 0.093 |

*high background
**low sensitivity

The Control A test device and the third test device (and respective reagent compositions) were used in assays again after keeping all materials at 25°C and less than 45% relative humidity for eight weeks. The results are shown below in Table II as the average of three replicates for the detection of 50 ml.U./ml hCG. Although the Control device and composition provided low background, they also gave low sensitivity. Only the composition and assay of this invention was acceptable in both characteristics.

TABLE II

| Assay | $D_T$ | |
|---|---|---|
| | Background | Test Well |
| Control A Example 4 | 0.012 0.011 | 0.054 0.076 |

Example 5: Use of Various Buffers in the Enzyme-Labeled Antibody Compositions

The reagent compositions of Examples 1-3 were used in an assay for hCG like that shown in Example 4. The results of the assays indicate that each reagent composition, after keeping tests, exhibited adequate to excellent sensitivity with low background.

**Claims**

1. An aqueous reagent composition having a pH of from 7 to 9 and comprising an enzyme-labeled receptor which is specifically reactive with a ligand of interest, the composition characterized as further comprising an organic buffer having a pKa of from 5 to 7 at 25° C and present in an amount of from 0.1 to 1 molar.

2. The composition as claimed in claim 1 wherein the receptor is an antibody.

3. The composition as claimed in claim 2 wherein the antibody is directed to hCG and is labeled with peroxidase.

4. The composition as claimed in any of claims 1 to 3 wherein the buffer has a pKa of from 5.5 to 6.5 at 25° C and is present in an amount of from 0.1 to 0.5 molar.

5. The composition as claimed in any of claims 1 to 4 wherein the buffer is 2-(N-morpholino)-ethanesulfonic acid.

6. A diagnostic test kit useful for the determination of a ligand comprising:
   a. the aqueous reagent composition as claimed in any of claims 1 to 5, and
   b. a dye-providing composition which provides a dye in the presence of the enzyme and a substrate therefor.

7. The kit as claimed in claim 6 wherein the receptor is an antibody and the kit further comprises a second antibody directed to a different epitopic site of the ligand, the second antibody being bound or capable of being bound to an insoluble support material.

8. The use of the aqueous reagent composition as claimed in any of claims 1 to 5.

9. A method for the determination of a ligand comprising:
   A. contacting a specimen suspected of containing a ligand of interest with an enzyme-labeled receptor therefor to form an immunological complex, the receptor provided in an aqueous reagent composition as claimed in any of claims 1 to 5,
   B. separating the complex from uncomplexed enzyme-labeled receptor, and
   C. detecting either the complex or uncomplexed enzyme-labeled receptor as an indication of the presence of the ligand in the specimen.

10. The method as claimed in claim 9 wherein the ligand is also complexed with a second receptor which is bound to or capable of being bound to an insoluble support material.

11. The method as claimed in claim 9 wherein the immunological complex is insolubilized and separated from uncomplexed materials using a microporous membrane in a disposable test device.

12. A method for the determination of hCG comprising:
   A. contacting a biological specimen with an unlabeled monoclonal antibody to hCG to form an immunological complex with any hCG present in the specimen,
   B. prior to, simultaneously with or subsequent to the contacting in step A, contacting the specimen with an aqueous reagent composition having a pH from 7 to 9 and comprising an enzyme-labeled monoclonal antibody to hCG in an organic buffer having a pKa of from 5 to 7 at 25° C and present in an amount of from 0.1 to 0.5 molar, the labeled antibody being directed to a different epitopic site than the unlabeled antibody,

C. separating the resulting complex of hCG and the antibodies from uncomplexed materials, and

D. adding a dye-providing composition which will provide a dye in the presence of the enzyme label as an indication of the presence of hCG in the specimen.

13. The method as claimed in claim 12 for the determination of hCG in urine.

14. The method as claimed in any of claims 9 to 13 which is carried out in a disposable test device comprising a microporous membrane for separating the complex from uncomplexed materials.